(19) Europäisches Patentamt
European Patent Office
Office européen des brevets

(11) EP 4 548 838 A1

(12) EUROPEAN PATENT APPLICATION

(43) Date of publication:
07.05.2025 Bulletin 2025/19

(21) Application number: 23386107.9

(22) Date of filing: 30.10.2023

(51) International Patent Classification (IPC):
A61B 5/02 (2006.01)

(52) Cooperative Patent Classification (CPC):
A61B 5/02007; A61B 6/481; A61B 6/5217;
A61B 6/504

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Medlytic Labs
26222 Patras (GR)

(72) Inventors:
• BEKIRIS, Fivos
26442, Patra (GR)
• BOURANTAS, Georgios
30131, Agrinio (GR)
• KATSANOS, Konstantinos
26442, Patra (GR)

(74) Representative: Dodou, Evdokia
Katsimpiri 3
15561 Cholargos, Athens (GR)

(54) **COMUTER-IMPLEMENTED METHOD AND SYSTEM FOR CALCULATING A PRESSURE DROP ALONG VESSELS**

(57) A computer-implemented method and a system for calculating a pressure drop along one or more vessels comprising one or more stenosis, the method comprising: obtaining at least one two-dimensional image of the vessels; obtaining a centerline and a boundary of each vessel by image processing algorithms; calculating a length and a diameter of each vessel; calculating a baseline volumetric flow ($Q_b$); deriving a hyperaemic volumetric flow rate ($Q_h$); determining a set of stenosed parts; defining a proximal part, between an inlet and a first stenosed part, a distal part, between last stenosed part and outlet, and a set of normal parts, between two stenosed parts; calculating a pressure drop at a proximal part, at a distal part, at normal parts and at stenosed parts, and calculating a total pressure drop $\Delta P$ from inlet to outlet of the vessel. Also, calculating resistance indices of the stenosis and of the microcirculation.

FIG. 6

**Description**

## DESCRIPTION OF THE INVENTION

It is an object of the present invention to provide a computer-implemented method for calculating a pressure drop along one or more coronary vessels, thus modeling the coronary physiology of a patient.

## OBJECT OF THE INVENTION

The present invention relates to the field of two-dimensional medical imaging, and in particular, to the field of coronary imaging with X-Ray angiography or computed tomography or magnetic resonance angiography.

The invention relates to a computer-implemented method for calculating a pressure drop along one or more coronary vessels, or any other vessels like the arteries of the lower limbs or the brain, and allowing to obtain a fractional flow reserve (FFR) and index of microvascular resistance (IMR) in the vessel.

Other object of the invention is a system for calculating a pressure drop along one or more coronary vessels, or any other vessels that may be imaged with two-dimensional techniques, following the steps of the method of the invention.

## BACKGROUND ART

Atherosclerosis produces stenoses in the coronary arteries that impede blood flow and oxygen delivery to the myocardium, resulting in ischemic coronary artery disease (CAD). Atherosclerosis also produces ischemic symptoms in the legs, aka peripheral artery disease or the brain, aka cerebrovascular disease. Hemodynamic factors play an important role not only in the development and progression of arterial stenoses, but also contribute to determining their physiological significance. The physiological significance of a stenosis cannot be judged solely based on its geometrical characteristics, but rather in association to given flow conditions.

In fact, mean pressure drop across a stenosis increases non-linearly with percent area stenosis and with increasing flow rates. Coronary angiography (in the form of X-Ray angiography or computed tomography or magnetic resonance angiography) allows for evaluation of the anatomical characteristics of stenoses of vascular geometries but is often inaccurate in identifying functionally significant coronary artery stenoses.

Modalities for physiological assessment of coronary artery disease may be applied non-invasively outside the catheterization laboratory for diagnostic and screening purposes, or inside the catheterization laboratory during coronary catheterization either to decide the need for invasive treatment, or to control and optimize the results of transcatheter coronary angioplasty and stenting.

Fractional Flow Reserve (FFR) is a hemodynamic index that quantifies the functional severity of a coronary artery stenosis. FFR was first introduced by N H Pijls, J A van Son, R L Kirkeeide, B De Bruyne and K L Gould in the paper entitled "Experimental basis of determining maximum coronary, myocardial, and collateral blood flow by pressure measurements for assessing functional stenosis severity before and after percutaneous transluminal coronary angioplasty" (Circulation 87(4) 1993, pp. 1354-1367) and is defined as the maximum achievable blood flow in the presence of a stenosis divided by maximum flow in the absence of any obstructive epicardial coronary disease at peak hyperaemia. Alternatively, the Hyperaemic Stenosis Resistance (HSR), which is defined as the ratio of the pressure drop and blood flow velocity across the interrogated vessel stenosis at peak hyperaemia, has been proposed as a useful index to quantify hemodynamic significance of the stenosis. FFR can be determined as the ratio of the pressure distal to the stenosis ($P_d$) to the pressure in the aorta ($P_a$) under conditions of peak hyperaemia achieved by intravenous or intra-arterial pharmacological agents.

Hemodynamic significance of vascular stenoses depends on the inter-related effects of reduced blood flow and microvascular resistance of target tissues. Abnormal FFR values capture the effect of proximal coronary artery stenosis in diminishing distal myocardial perfusion at peak hyperaemia, with cut-off values of $\leq 0.80$ being well accepted as the threshold for stent placement or other kind of invasive revascularization.

The guidelines recommend functional imaging by MRI or SPECT or coronary CT for diagnosis of suspected chronic coronary syndromes. FFR-CT has been introduced over the last decade to address the moderate specificity of coronary CT angiography (CCTA) and incorporates three-dimensional (3D) reconstruction of the coronary arteries followed by computational fluid dynamics (CFD) to derive hyperaemic pressure ratios under certain boundary conditions and physical assumptions. Addition of functional FFR-CT on top of standard anatomical CCTA has shown the same sensitivity, but significantly higher specificity for diagnosis of CAD (71% versus 32%).

Suboptimal image quality because of irregular cardiac rhythm, motion artefacts or obesity and vessel calcification that prohibits accurate delineation of the vessel lumen remain the major limitations of current FFR-CT approaches with reported rejection rates ranging from 3.9% to 13%.

Inside the catheterization laboratory, invasive wire-based FFR has been used for physiological assessment when severity of stenosis ranges between 50% and 90% diameter stenosis by visual estimate, but myocardial ischemia remains unconfirmed.

There is now strong evidence from multiple studies that FFR-guided percutaneous coronary intervention (PCI) is associated with significantly lower long-term risk of death, lower risk of myocardial infarction, and lower risk of repeat coronary revascularization compared to an angiography-guided PCI strategy under several different clinical scenarios. Hence, current guidelines recommend the use of FFR in identifying functionally significant epicardial coronary artery stenoses and determining the need for PCI.

However, wire-based methods for FFR measurement suffer from a number of pitfalls and limitations restricting their use and applicability to a minority of eligible cases.

First, they mandate the administration of pharmacological vasodilators that produce patient discomfort during injection. Second, wire-based FFR increase the duration of the procedure, and are inherently invasive and costly to implement. In addition, poor quality of the pressure guidewires may render difficulties and complications during wire manipulation across complex lesions. Finally, almost one-third of FFR tracings suffer from either significant drift or waveform abnormalities, diminishing the quality of the measurements. Overall, current FFR utilization rates in assessing intermediate stenoses remain disappointingly low at less than 20% of the relevant cases.

FFR allows for physiological assessment only of obstructive disease of the major epicardial vessels with diameter >0.5 millimeters. Disease state and function of the microcirculation (arterioles and capillaries with diameter <100 micrometers that are below the resolution of current angiography systems) can be indirectly probed and assessed by changes in macrovascular flow and velocity patterns.

Microvascular dysfunction leads to impaired distribution of blood flow and regulation of the tissue metabolism. Consequently, the absolute Microvascular Resistance (MVR) and the Index of Microvascular Resistance (IMR) have evolved as measures of disturbances and increased flow resistance of the coronary microcirculation and can predict poor outcomes in patients who have suffered a myocardial infarction. The aforementioned IMR and MVR mandate the use of dedicated coronary wires equipped with Doppler capacity or thermodilution techniques.

Contrary to invasive wire-based methods, image-based methods have been also developed to compute FFR and IMR utilizing computer models of blood pressures and flow into the coronary arteries using information of vessel anatomy and morphology derived from routine angiographic images. However, they are complex, time-consuming and typically rely on the analysis of three-dimensional images that require extra acquisition, analytical and computational steps.

Modern applications of FFR and IMR for physiologically guided coronary interventions include first, baseline interrogation of the hemodynamic significance of arterial stenoses to avoid unnecessary revascularization procedures, second improving procedural outcomes by incorporating functional post-treatment assessment or even side-branch interrogation, and third, diagnosing microvascular disease and dysfunction that predict adverse long-term outcomes in patients with myocardial infarction.

Examples of existing methods used for calculating the fractional flow reserve (FFR) include:

Method 1: measuring the pressure ($P_d$) at the stenosed distal end of the coronary artery in the myocardium's maximum hyperaemia state by a pressure guide wire, thereby calculating the FFR. There are numerous pressure wires currently commercially available for the invasive measurement of FFR and/or IMR in the coronary arteries. IMR is routinely measured with thermodilution. Both FFR and IMR require induction of pharmacological vessel hyperaemia.

Method 2: acquiring a three-dimensional morphology of blood vessels based on a coronary angiography image, calculating blood flow and calculating the mean intracoronary pressure ($P_d$) at the stenosed distal end by computational fluid dynamics (CFD) simulation to calculate the FFR (in the European Patent Application EP 3 763 285 A1 of Suzhou Rainmed Medical Technology Co., Ltd with the title *"METHOD FOR MEASURING FRACTIONAL FLOW RESERVE WITHOUT VASODILATOR"*).

Method 3: receiving a plurality of angiographic images of a portion of a vasculature of a subject at different projection angles (in the U.S. Published Patent Application No. 9,858,387 B2 of CathWorks Ltd, Tel Aviv with the title *"VASCULAR FLOW ASSESSMENT"*), and processing the images to produce a three-dimensional morphology of a stenotic model over the vasculature, the stenotic model having measurements of the vasculature at one or more locations along vessels of the vasculature. The method, in some embodiments, further comprises obtaining a flow characteristic of the stenotic model, and calculating an index indicative of vascular function, based, at least in part, on the flow characteristic in the stenotic model to calculate the FFR.

Method 4: receiving a plurality of angiographic images of a portion of a vasculature of a subject at different projection angles (in the paper Huo Y, Svendsen M, Choy J S, Zhang Z.-D and Kassab G S entitled "A validated predictive model of coronary fractional flow reserve"(J. R. Soc. Interface (2012) 9, 1325-1338)), deriving a patient specific geometric model of patient's blood vessels, and combing this geometry with the patient-specific physiological information and boundary conditions to estimate blood flow characteristics and predict clinically relevant quantities of interest (e.g. FFR).

Method 5: receiving a plurality of angiographic images of a portion of a vasculature of a subject at different projection angles (as in WIPO/PCT patent with International Publication Number WO 2019/238754 A1 of PIE MEDICAL IMAGING BV with the title *"METHOD AND APPARATUS FOR QUANTITATIVE HEMODYNAMIC FLOW ANALYSIS"*),

deriving a patient specific geometric model of patient's blood vessels, and computing pressure drop along the stenotic part of the vessel using mathematical modelling.

The FFR in the above methods is calculated based on a blood flow velocity and the mean aortic pressure ($P_a$) at the coronary artery inlet in the myocardium's maximum hyperaemia state, and the mean intracoronary pressure ($P_d$) at the stenosed distal end. Additionally, computations take place in a 3D reconstructed geometry of the coronary artery.

Image-based computer methods cannot reliably simulate hyperaemia and are uniformly time-consuming in the order of several minutes per image-reconstruction and FFR calculation, which intuitively is not clinically applicable and relevant into patient throughput of modern catheterization laboratories.

Moreover, currently available medical software for FFR measurement is not fully automatic and require different levels of user interaction at different steps.

Currently available image-based computer methods routinely rely on a three-dimensional reconstruction of the coronary arterial tree (or the interrogated vessel if the analysis applies to only one of the vessels). This step involves a rudimentary application of epipolar geometry principles on two separate angiographic images with >25° angle difference and may suffer from several errors and pitfalls during image acquisition, image registration and subsequent segmentation of vascular geometries. Three-dimensional analyzability for image based FFR computations has been shown not to exceed 70-80% of the cases.

## DETAILED DESCRIPTION OF THE INVENTION

It is an object of the present invention to provide a computer-implemented method for calculating a pressure drop along one or more coronary vessels, thus modeling the coronary physiology of a patient.

The method of the invention allows to compute a pressure drop and a coronary angiography fractional flow reserve (FFR) and index of microvascular resistance (IMR) in a coronary artery or any other artery by using routine two-dimensional X-ray coronary angiography, or computed tomography or magnetic resonance angiography, an aortic pressure and blood flow images, without vasodilator medications (i.e., without the maximum hyperaemia state of the myocardium and in the absence of adenosine); detecting myocardial ischemia in patients with coronary heart disease.

The computer-implemented method of the invention allows to calculate a pressure drop along one or more coronary vessels comprising one or more stenosis. For that, the method of the invention comprises a step of obtaining at least one two-dimensional image of the coronary vessels. Preferably, a plurality of two-dimensional images is obtained and one of them is selected for the method of the invention. There is no need to combine different images at different angles for production of three-dimensional vessel geometries.

The two-dimensional image could be obtained from X-Ray angiography, computed tomography or magnetic resonance angiography.

Using the obtained two-dimensional image, a centerline and a boundary of each coronary vessel is determined by using image processing algorithms. Preferably, a vessel delineation algorithm, such as algorithms using artificial intelligence and/or deep learning.

Then, the method comprises a step of calculating a length, a diameter and a volume of each coronary vessel by using the centerline and the boundary obtained.

A baseline volumetric flow ($Q_b$) is obtained at rest conditions (without hyperaemia). The volumetric flow ($Q$) could be a volumetric flow rate at hyperaemia ($Q_h$). Hyperaemic flow conditions could be simulated based on prior experimental art. In the paper Gould, K Lipscomb and G W Hamilton entitled "Physiologic basis for assessing critical coronary stenosis. Instantaneous flow response and regional distribution during coronary hyperemia as measures of coronary flow reserve" (The American Journal of Cardiology 33(1) 1974 pp. 87-94) authors studied the effect of coronary stenosis on coronary flow reserve, i.e. studied coronary blood flow at rest and the maximal achievable flow at hyperaemia in animal experiments. This study states that the volumetric flow rate ($Q_h$) at hyperaemia could be obtained by calculating the coronary flow reserve (CFR) given by Gould's formula:

$$CFR = 3.7 - 0.01x + 0.00024x^2 - 0.000006x^3$$

wherein $x$ is the stenosis percentage defined as the ratio of the minimum diameter in the stenosed parts over the reference sizing i.e the healthy lumen as if there was no stenosis, and then, defining the volumetric flow rate ($Q_h$) at hyperaemia as:

$$Q_h = CFR \times Q_b$$

Thus, the volumetric flow rate at hyperaemia ($Q_h$) could be calculated by following the stages of:

- calculating a baseline volumetric flow ($Q_b$);
- obtaining a minimum diameter in the stenosed parts;
- calculating a coronary flow reserve (CFR) as:

$$CFR = 3.7 - 0.01x + 0.00024x^2 - 0.000006x^3$$

wherein $x$ is the stenosis percentage defined as the ratio of the minimum diameter in the stenosed parts over the reference sizing i.e the healthy lumen as if there was no stenosis;

- calculating the volumetric flow rate at hyperaemia ($Q_h$) as:

$$Q_h = CFR \times Q_b$$

For calculating the baseline volumetric flow ($Q_b$) some different methods could be used.

A reference method implies that the two-dimensional image is obtained from X-Ray angiography. Then, a volume of each coronary vessel is calculated by using the length and diameter of the vessel. Then, approximating a crossing time ($t_c$), by using a TIMI Frame Count (TFC) and the angiographic acquisition rate (frames per second (fps)) as:

$$t_c = \text{TFC/fps}$$

The baseline volumetric flow ($Q_b$) is calculated by using the volume of each coronary vessel $V_{vessel}$ and the crossing time as:

$$Q_b = V_{vessel}/t_c$$

The velocity of an iodinated contrast is computed by measuring a vessel length and a time (crossing time $t_c$) taken to cross said vessel length.

A second method for calculating the baseline volumetric flow ($Q_b$) comprises the steps of:

- calculating a volume ($V_{vessel}$) of each coronary vessel by using the length ($L_{vessel}$) and diameter;
- setting a constant velocity ($U$) for the blood flow in the coronary artery, preferably in the range of 0.14 - 0.16 m/s;
- computing a crossing time ($t_c$) using the formula:

$$t_c = L_{vessel}/U$$

Then, the baseline volumetric flow ($Q_b$) is calculated by using the volume $V_{vessel}$ of each coronary vessel and the crossing time ($t_c$) as:

$$Q_b = V_{vessel}/t_c$$

A third method for calculating the baseline volumetric flow ($Q_b$) is based on the fact that volumetric flow rates may be predicted on the basis of well-known laws of allometric scaling that are known (since 1932) to govern mammalian biological systems. Human and animal experiments have shown that coronary flow is directly related to coronary luminal volume. In addition, myocardial mass and coronary flow scale to certain power-law relationships with respect to coronary artery morphometry like for example diameter and length of the coronary arteries. Therefore, the baseline volumetric flow ($Q_b$) may be obtained by power-law formulae in the general form of

$$Q = aX^b$$

wherein X is a morphological parameter (i.e. vessel length, vessel volume, vessel lumen area) extracted from the two-dimensional angiographic images (diameter, length or axisymmetric volume), *a* is a constant calculated by fitting regression models on previously analyzed experimental or human data, and *b* is a fitted power-law exponent based on previously analyzed experimental or human data, which preferably is in the range of 0.6 - 0.8, and more preferably in the range of 0.65 - 0.70.

This third method could be used instead of measuring TFC in X-Ray angiography sequences, or in the absence of such information like in the case of computed tomography coronary imaging or magnetic resonance angiography that lack time-resolved image acquisitions.

In a fourth alternative way, the hyperaemic volumetric flow ($Q_h$) could be calculated by following the stages of:

- fixing the hyperaemic flow velocity as a constant value in the range of

$$u_h = 0.25 - 0.45 \ m/s;$$

- calculating a volume ($V_{vessel}$) of each coronary vessel by using the length ($L_{vessel}$);
- computing a crossing time ($t_c$) needed by a contrast medium to cross from an inlet to an outlet of the vessel as:

$$t_c = \frac{L_{vessel}}{u_h}$$

- calculating the hyperaemic volumetric flow rate as:

$$Q_h = \frac{V_{vessel}}{t_c}$$

Then, a set of normal and a set of stenosed parts are determined. Also, a proximal part of the vessel is defined as the segment between an inlet and a first stenosed part. In the same way, a distal part of the vessel is defined as a segment between a last stenosed part and an outlet. Then, a set of normal parts is determined as the segments between any two stenosed parts.

Then, a pressure drop at a proximal normal part and a pressure drop at a distal normal part are calculated as:

$$\Delta p_p = \frac{8\pi\mu L_p}{A_{p,ref}^2} Q_h$$

$$\Delta p_d = \frac{8\pi\mu L_d}{A_{d,ref}^2} Q_h$$

wherein $A_{p,ref}$ is a reference cross-sectional area at the proximal part of the vessel, $A_{d,ref}$ is a reference cross-sectional area at the distal part of the vessel, $L_p$ is a length of the proximal part of the vessel and $L_d$ is a length of the distal part of the vessel.

Also, a pressure drop at normal parts and a pressure drop at stenosed parts are calculated, respectively, as:

$$\Delta p_n = \frac{8\pi\mu L_n}{A_{n,ref}^2} Q_h$$

$$\Delta p_s = \frac{8\pi\mu L_s}{A_s^2} Q_h + \frac{1.52}{2} \rho \left( \frac{1}{A_s} - \frac{1}{A_{p,ref}} \right)^2 Q_h^2$$

wherein $A_{n,ref}$ is a reference cross-sectional area at the normal part of the vessel, $L_n$ is a length of the normal part of the vessel, $A_{p,ref}$ is a reference cross-sectional area at the proximal part of the vessel, $A_s$ is a minimum cross-sectional area at the stenosed part of the vessel and $L_s$ is a length of the stenosed part of the vessel.

Then, a total pressure drop $\Delta P$ from the inlet to the outlet of the vessel is calculated using the formula:

$$\Delta P = \Delta p_p + \sum_{i=1}^{m} \Delta p_s + \sum_{j=1}^{m-1} \Delta p_n + \Delta p_d$$

Also, a fractional flow reserve (FFR) could be calculated using different processes. A first process for calculating the fractional flow reserve (FFR) comprises the step of obtaining a pressure at the inlet ($P_a$), which is equal to the mean aortic pressure (MAP) in rest state ($P_a$=MAP). Then, a pressure at the distal part ($P_d$) is calculated as:

$$P_d = P_a - \Delta P$$

The fractional flow reserve (FFR) is then calculated as:

$$FFR = P_d/P_a$$

In a second process for calculating the fractional flow reserve (FFR), the steps of calculating a pressure drop at a proximal part and a pressure drop at a distal part, calculating a pressure drop at normal parts and a pressure drop at stenosed parts and calculating a total pressure drop $\Delta P$ are performed twice, assigning a first volumetric flow ($Q_1$) and obtaining a first total pressure drop $\Delta P_1$, and assigning a second volumetric flow ($Q_2$) and obtaining a second total pressure drop $\Delta P_2$.

It is well-established that pressure loss across arterial stenosis is well-described by quadratic equations that describe pressure drops as the sum of viscous, turbulent and inertia contributions.

$$R = \frac{\Delta p}{Q} = a_v + b_v Q$$

In this approach, resistance is defined as and pressure drop is defined as $\Delta p = a_v Q + b_v Q^2$, which is a well known formulation that govern blood flow across vascular stenoses.

The aforementioned quadratic relationships between blood flow and pressure drop have been quite successful in predicting experimental data in short axi-symmetrical and non-symmetrical stenoses under steady flow conditions of Newtonian fluids.

Therefore, the total pressure drop $\Delta P$ is defined as:

$$\Delta P = a_v Q + b_v Q^2$$

Parameters ($a_v$, $b_v$) are obtained by solving the equation system:

$$\Delta P_1 = a_v Q_1 + b_v Q_1^2$$

$$\Delta P_2 = a_v Q_2 + b_v Q_2^2$$

Also, the ratio $P_d/P_a$ is calculated as:

$$\frac{P_d}{P_a} = 1 - \frac{a_v}{P_a}Q + \frac{b_v}{P_a}Q^2$$

Then, the ratio $P_d/P_a$ is plotted versus the flow Q in the coronary artery up to flow value 4ml/s and the FFR is calculated by dividing the area-under-curve of the aforementioned plot by the maximum flow value 4.

In a preferred embodiment, the first volumetric flow ($Q_1$) is set to 1 mils and the second volumetric flow ($Q_2$) is set to 3 mils. In another preferred embodiment, the first volumetric flow ($Q_1$) is set to 1 mils and the second volumetric flow ($Q_2$) is set equal to CFR mils.

The method of the invention could also comprise a step of calculating a hyperaemic stenosis resistance (HSR), an absolute microvascular resistance and an index microvascular resistance (IMR) as follows, respectively:

$$HSR = \frac{\Delta P}{Q_h} \text{ (divided by flow Q) or alternatively } HSR = \frac{\Delta P}{U} \text{ (divided by Velocity U)}$$

$$MVR = \frac{Pa - \Delta P}{Q_h} \text{ or } MVR = \frac{Pd}{Q_h}, IMR = \frac{Pa - \Delta P}{CFR} \cdot t$$

Aforementioned parameters are typically calculated at peak hyperaemia using Gould's CFR.

The invention also relates to a system for calculating a pressure drop along one or more coronary vessels. The system of the invention comprises:

- an X-Ray angiography device, computed tomography device or magnetic resonance angiography device for obtaining at least one two-dimensional image of the coronary vessels; and
- a processing unit configured for performing the steps of the method previously defined.

## DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:

Figure 1.- schematically illustrates a drawing of the coronary or other artery and its respective microcirculation and demonstrates the different indexes (CFR, FFR, MVR, IMR) that may be computed with the proposed invention using any 2D angiographic image and measuring transit time t, vessel volume V and flow rate Q according to the embodiments of the invention.

Figure 2.- illustrates an example of a 2D angiographic image (101) showing a coronary vessel tree, and a vessel (10) according to an embodiment of the invention.

Figure 3.- illustrates a geometrical model of a vessel (10) obtained from a 2D angiographic image (101) for assessing a patient's vasculature according to an embodiment of the invention.

Figure 4.- illustrates an example of a vessel (10) with the vessel boundaries (31) defined (solid line) and the centerline (30) (dashed line) according to an embodiment of the invention.

Figure 5.- illustrates the radius of the vessel along the centerline (solid line) and the radius of the vessel along the centerline after extrapolation of the expected normal diameter (dashed line) according to an embodiment of the invention.

Figure 6.- illustrates a block-diagram of the pipeline of an embodiment of the method of the invention.

## PREFERRED EMBODIMENTS OF THE INVENTION

A set of preferred embodiments of the invention are presented and illustrated by the accompanying figures.

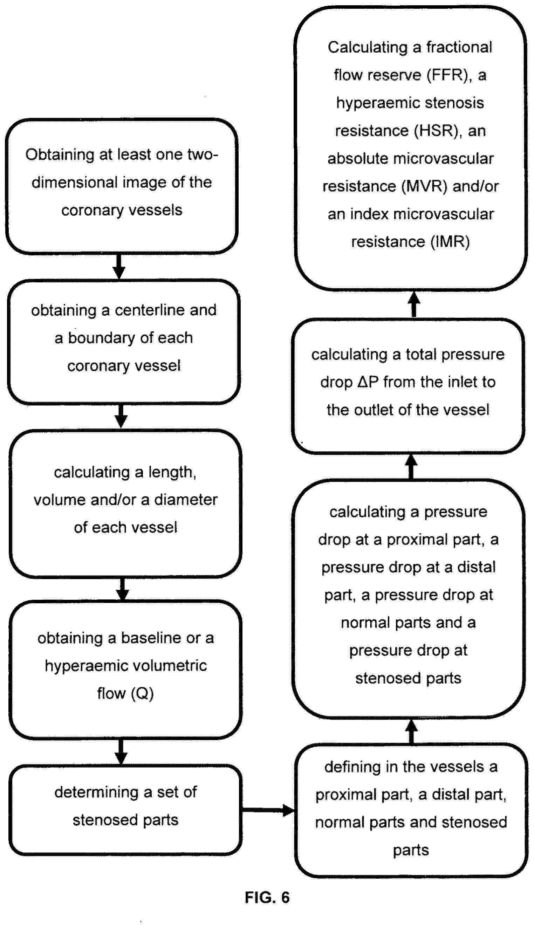

In particular, figure 6 shows a set of steps common to the embodiments of the present invention.

A first embodiment relates to a method for computing a pressure drop and a fractional flow reserve (FFR) in a vessel without using a vasodilator.

In this embodiment, the method of the invention comprises the steps of:

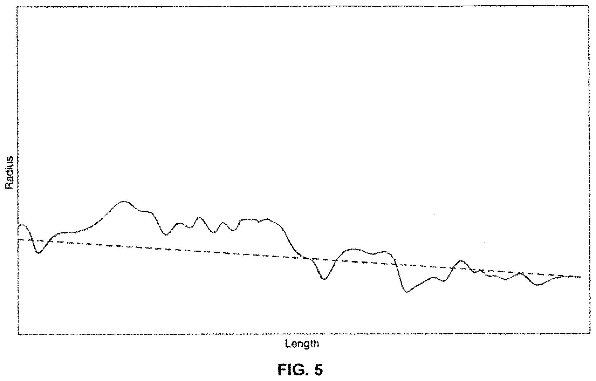

- Acquiring a single diagnostic image (101) and/or a plurality of diagnostic images of the vasculature, as time series, by using X-ray angiography.
- Extracting one two-dimensional angiographic image (101) of the vasculature from the time series of diagnostic images.
- In the single two-dimensional angiographic image (101) applying vessel delineation and calculating boundaries (31) of the vessel (10), then, computing a vessel centerline (30); and computing a length and a diameter of a vessel (10) through the centerline (30) and the vessel boundaries (31), resulting in a graph as the one shown in Figure 5, respectively. Then, a volume ($V_{vessel}$) of the vessel (10) is calculated.
- Defining stenosed parts (12) (m in total) of the vessel (10). The vessel part between the inlet (15) and the first stenosed part (12) is determined as the proximal part (18) of the vessel (10). The vessel part between the end/outlet (13) of the last stenosed part (12) and the end or outlet of the vessel (10) is determined as the distal part (16) of the vessel (10). The remaining parts are determined as normal parts (22).
- Then, computing in each stenosed part (12) of the vessel (10) the minimum diameter, the maximum diameter, and the mean diameter; also computing in the proximal part (18) and the distal part (16) of the vessel (10) the minimum diameter, the maximum diameter and the mean diameter; and computing in the normal parts (22) of the vessel (10) the

8

minimum diameter, maximum diameter and the mean diameter.

- Then, measuring a length of the vessel (10), and obtaining a crossing time ($t_c$) from inlet (15) to outlet (13) and a baseline volumetric flow rate ($Q_b$), based on the crossing time and the volume ($V_{vessel}$) of the vessel (10), by following the stages of:

  • acquiring an image acquisition rate (fps) from angiography image information;
  • computing a crossing time ($t_c$), using a TIMI Frame Count (TFC) method, through which the blood flows from an input landmark location or inlet (15) to an output landmark location or outlet (13) of the vessel (10), and wherein the crossing time ($t_c$) is computed as:

$$t_c = \frac{TFC}{fps}$$

  • obtaining the baseline volumetric flow rate ($Q_b$) using the volume of the vessel (10) ($V_{vessel}$) and the crossing time ($t_c$) using the formula:

$$Q_b = V_{vessel}/t_c$$

- Calculating a coronary flow reserve (CFR), computed by using a Gould's formula as:

$$CFR = 3.7 - 0.01x + 0.00024x^2 - 0.000006x^3$$

wherein $x$ is the stenosis percentage, defined as the ratio of the minimum diameter in the stenosed parts (12) of vessel (10) over the reference sizing i.e. the healthy lumen as if there was no stenosis.

- Calculating a volumetric flow rate ($Q_h$) at hyperaemia as:

$$Q_h = CFR \times Q_b$$

- Calculating a pressure drop ($\Delta p_p$) at a proximal part (18) of the vessel (10) using the Hagen-Poiseuille equation:

$$\Delta p_p = \frac{8\pi\mu L_p}{A_{p,ref}^2} Q_h$$

wherein $A_{p,ref}$ is a reference cross-sectional area at the proximal part (18) of the vessel (10), calculated by diving the axisymmetric volume of the proximal segment of the vessel (10) by its respective length $L_p$, and $L_p$ is the length of the proximal part (18) of the vessel (10).

- Calculating a pressure drop ($\Delta p_d$) at a distal part (16) of the vessel (10) using the Hagen-Poiseuille equation:

$$\Delta p_d = \frac{8\pi\mu L_d}{A_{d,ref}^2} Q_h$$

wherein $A_{d,ref}$ is a reference cross-sectional area at the distal part (16) of the vessel (10), calculated by diving the axisymmetric volume of the distal segment of the vessel (10) by its respective length $L_d$, and $L_d$ is the length of the proximal part (18) of the vessel (10).

- Calculating a pressure drop ($\Delta p_n$) at a normal part (22) of the vessel (10) using the Hagen-Poiseuille equation:

$$\Delta p_n = \frac{8\pi\mu L_n}{A_{n,ref}^2} Q_h$$

wherein $A_{n,ref}$ is a reference cross-sectional area at the normal part (22) of the vessel (10), calculated by diving the axisymmetric volume of the normal segment of the vessel (10) by its respective length $L_n$, and $L_n$ is the length of the normal part (22) of the vessel (10).

- Calculating a pressure drop ($\Delta p_s$) at a stenosed part (12) of the vessel (10) using the equation:

$$\Delta p_s = \frac{8\pi\mu L_s}{A_s^2} Q_h + \frac{1.52}{2} \rho \left( \frac{1}{A_s} - \frac{1}{A_{p,ref}} \right)^2 Q_h^2$$

wherein $A_{p,ref}$ is a reference cross-sectional area at the proximal part (18) of the vessel (10), calculated by diving the axisymmetric volume of the proximal segment of the vessel (10) by its respective length $L_p$, $A_s$ is the minimum cross-sectional area at the stenosed part (12) of the vessel (10), and $L_s$ is the length of the stenosed part (12) of the vessel (10).

- Calculating a pressure drop ΔP from the vessel inlet (15) to a distal end or outlet (13) of a vessel (10) using the formula:

$$\Delta P = \Delta p_p + \sum_{i=1}^{m} \Delta p_s + \sum_{j=1}^{m-1} \Delta p_n + \Delta p_d$$

- Calculating a mean aortic pressure (MAP) in the rest state, defined as a pressure ($P_a$) at the vessel inlet (15).
- Calculating a mean intracoronary pressure ($P_d$) at the distal end or outlet (13) of the vessel (10), by using the formula:

$$P_d = P_a - \Delta P$$

- Calculating a coronary angiography fractional flow reserve (FFR) using the formula:

$$FFR = P_d/P_a$$

In a second embodiment of the invention the hyperaemic volumetric flow rate ($Q_h$) is calculated by:

- obtaining a constant velocity ($U$=0.25 -0.45 m/s) for the blood flow in the vessel (10);
- obtaining a vessel length ($L_{vessel}$) of the vessel (10);
- computing the crossing time ($t_c$) using the formula:

$$t_c = L_{vessel}/U$$

- calculating a hyperaemic volumetric flow rate ($Q_h$) based on the time and the volume ($V_{vessel}$) of the vessel (10) using the formula:

$$Q_h = V_{vessel}/t_c$$

A pressure drop ΔP and a coronary angiography fractional flow reserve (FFR) are computed using the formulas described in the first embodiment.

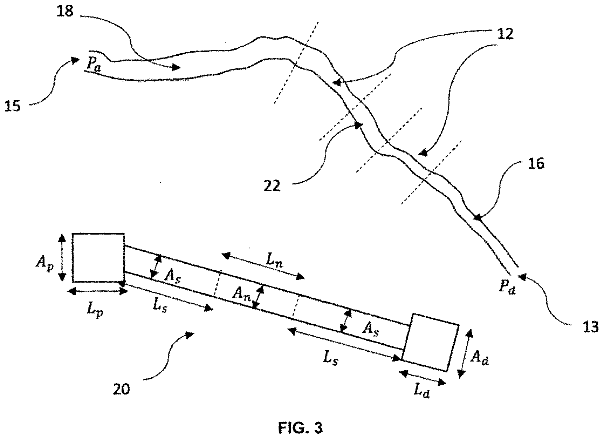

In a third embodiment of the invention, the blood flow velocity ($u$) in the vessel (10) is calculated by a numerical method, using a two-dimensional axisymmetric blood vessel model (20), shown in Figure 3, and an approximation for incompressible flow, in particular, a continuity equation and a Navier-Stokes equation:

$$\nabla \cdot u = 0$$

$$\rho \left( \frac{\partial u}{\partial t} + (u \cdot \nabla)u \right) = -\nabla p + \mu \nabla^2 u$$

wherein, u is the blood flow velocity, $p$ is the pressure, $\rho$ is a blood density, and $\mu$ is a blood viscosity; the mean arterial pressure ($P_a$) is a vessel inlet (15) boundary condition, and the computed hyperaemic volumetric flow rate ($Q_h$) (as in the previous embodiment) is a vessel outlet (13) boundary condition.

In a fourth embodiment, the method comprises the steps of:

- Acquiring a single two-dimensional X-Ray angiographic image (101).
- Applying computer algorithms for vessel delineation and calculating boundaries (31) of the vessel (10), then, computing a vessel centerline (30) and computing a length ($L_{vessel}$) and a diameter (shown in figure 5) of the vessel (10) through the centerline (30) and the vessel boundaries (31), respectively. Then, computing the volume ($V_{vessel}$) of the vessel (10).

- Defining stenosed parts (12) (m in total) of the vessel (10). The vessel part between the inlet (15) and the first stenosed part (12) is determined as the proximal part (18) of the vessel (10). The vessel part between the end of the last stenosed part (12) and the vessel end or outlet (13) of the vessel (10) is determined as the distal part (16) of the vessel (10). The remaining parts are determined as normal parts (22).

- Computing a reference diameter $D_{ref}$ using the formula:

$$D_{ref} = 2\sqrt{\frac{V_{vessel}}{\pi L_{vessel}}}$$

- Computing a reference radius $R_{ref}$ as:

$$R_{ref} = \frac{D_{ref}}{2}$$

- Calculating a pressure drop ($\Delta p_p$) at a proximal part of the coronary vessel using the Hagen-Poiseuille equation and assigning a volumetric flow rate of

$$Q_1 = 1\ mL/s:$$

$$\Delta p_p = \frac{8\pi\mu L_p}{A_{ref}^2} Q_1$$

wherein $A_{ref} = \pi R_{ref}^2$ and $L_p$ is a length of the proximal part (18) of the vessel (10).

- Calculating the pressure drop ($\Delta p_d$) at a distal part (16) of the vessel (10) using the Hagen-Poiseuille equation:

$$\Delta p_d = \frac{8\pi\mu L_d}{A_{ref}^2} Q_1$$

wherein $L_d$ is the length of the proximal part (18) of the vessel (10).

- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q_1$$

wherein $L_n$ is the length of the normal part (22) of the vessel (10).

- Calculating the pressure drop ($\Delta p_s$) at a stenosed part (12) of the vessel (10) using the equation:

$$\Delta p_s = \frac{8\pi\mu L_s}{A_s^2} Q_1 + \frac{1.52}{2}\rho\left(\frac{1}{A_s} - \frac{1}{A_{ref}}\right)^2 Q_1^2$$

wherein $A_s$ is the minimum cross-sectional area at the stenosed part (12) of the vessel (10) and $L_s$ is the length of the stenosed part (12) of the vessel (10).

- Calculating the pressure drop pressure-drop $\Delta P_1$ from the vessel inlet (15) to the end or outlet (13) of the vessel (10) using the formula:

$$\Delta P_1 = \Delta p_p + \sum_{i=1}^{m} \Delta p_s + \sum_{j=1}^{m-1} \Delta p_n + \Delta p_d$$

- Calculating the pressure drop ($\Delta p_p$) at a proximal part (18) of the vessel (10) using the Hagen-Poiseuille equation and assigning a volumetric flow rate of

$$Q_2 = 3 \ mL/s:$$

$$\Delta p_p = \frac{8\pi\mu L_p}{A_{ref}^2} Q_2$$

wherein $A_{ref} = \pi R_{ref}^2$ is the reference cross-sectional area at the proximal part (18) of the vessel (10), and $L_p$ is the length of the proximal part (18) of the vessel (10).

- Calculating the pressure drop ($\Delta p_d$) at a distal part (16) of the vessel (10) using the Hagen-Poiseuille equation:

$$\Delta p_d = \frac{8\pi\mu L_d}{A_{ref}^2} Q_2$$

wherein $L_d$ is the length of the distal part (16) of the vessel (10).

- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q_2$$

wherein $L_n$ is the length of the normal part (22) of the vessel (10).

- Calculating the pressure drop ($\Delta p_s$) at a stenosed part (12) of the vessel (10) using the equation:

$$\Delta p_s = \frac{8\pi\mu L_s}{A_s^2} Q_2 + \frac{1.52}{2} \rho \left( \frac{1}{A_s} - \frac{1}{A_{ref}} \right)^2 Q_2^2$$

wherein $A_s$ is the minimum cross-sectional area at the stenosed part (12) of the vessel (10) and $L_s$ is the length of the stenosed part (12) of the vessel (10).

- Calculating the pressure drop pressure-drop $\Delta P_2$ from the vessel inlet (15) to the end or outlet (13) of the vessel (10) using the formula:

$$\Delta P_2 = \Delta p_p + \sum_{i=1}^{m} \Delta p_s + \sum_{j=1}^{m-1} \Delta p_n + \Delta p_d$$

- Calculating coefficients $a_v$ and $b_v$ of a general form of pressure drop in the stenosis (12) expressed as a 2 × 2 linear system in the form:

$$\Delta P = a_v Q + b_v Q^2$$

$$\Delta P_1 = a_v + b_v$$

$$\Delta P_2 = 3a_v + 9b_v$$

- Plotting the pressure drop $\Delta P$ versus the volumetric flow rate $Q$, obtaining a $\Delta P$ - $Q$ curve.
- Calculating $P_d/P_a$ as:

$$\frac{P_d}{P_a} = 1 - \frac{a_v}{P_a} Q + \frac{b_v}{P_a} Q^2$$

- Plotting the ratio $P_d/P_a$ versus the flow Q in the coronary artery up to flow value 4ml/s and then calculating FFR by dividing the area-under-curve of the aforementioned plot by the maximum flow value 4.

A fifth embodiment of the invention is also presented, wherein the method of the invention comprises:

- Acquiring a single two-dimensional X-Ray angiographic image (101).
- Applying computer algorithms for vessel delineation and calculating boundaries (31) of the vessel (10), then, computing a vessel centerline (30); computing a length ($L_{vessel}$) and a diameter of a vessel (10) through the centerline (30) and the vessel boundaries (31), respectively; and, then, computing the volume ($V_{vessel}$) of the vessel (10).
- Defining stenosed parts (12) (m in total) of the vessel (10). The vessel part between the inlet (15) and the first stenosed part (12) is determined as the proximal part (18) of the vessel (10). The vessel part between the end of the last stenosed part (12) and the end or outlet (13) of the vessel (10) is determined as the distal part (16) of the vessel (10). The remaining parts are determined as normal parts (22).
- Computing a reference diameter $D_{ref}$ using the formula:

$$D_{ref} = 2\sqrt{\frac{V_{vessel}}{\pi L_{vessel}}}$$

- Computing a reference radius $R_{ref}$ as:

$$R_{ref} = \frac{D_{ref}}{2}$$

- Obtaining a minimum diameter $D_{min}$.
- Computing using the geometrical data, reference diameter $D_{ref}$ and the minimum diameter $D_{min}$, the coronary flow reserve (CFR) given by Gould's formula

$$CFR = 3.7 - 0.01x + 0.00024x^2 - 0.000006x^3$$

wherein $x$ is the stenosis percentage defined as the ratio of the minimum diameter in the stenosed parts (12) of vessel (10) over the reference diameter $D_{ref}$ of the vessel (10).
- Calculating a pressure drop ($\Delta p_p$) at a proximal part (18) of the vessel (10) using the Hagen-Poiseuille equation and assigning a volumetric flow rate of

$$Q_1 = 1 \, mL/s:$$

$$\Delta p_p = \frac{8\pi\mu L_p}{A_{ref}^2} Q_1$$

wherein $A_{ref} = \pi R_{ref}^2$ is a reference cross-sectional area of the vessel (10), and $L_p$ is a length of the proximal part (18) of the vessel (10).
- Calculating the pressure drop ($\Delta p_d$) at a distal part (16) of the vessel (10) using the Hagen-Poiseuille equation:

$$\Delta p_d = \frac{8\pi\mu L_d}{A_{ref}^2} Q_1$$

wherein $L_d$ is the length of the distal part (18) of the vessel (10).
- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q_1$$

wherein $L_n$ is the length of the normal part (22) of the vessel (10).
- Calculating the pressure drop ($\Delta p_s$) at a stenosed part (12) of the vessel (10) using the equation:

$$\Delta p_s = \frac{8\pi\mu L_s}{A_s^2} Q_1 + \frac{1.52}{2} \rho \left( \frac{1}{A_s} - \frac{1}{A_{ref}} \right)^2 Q_1^2$$

wherein $A_s$ is the minimum cross-sectional area at the stenosed part (12) of the vessel (10) and $L_s$ is the length of the stenosed part (12) of the vessel (10).
- Calculating the pressure drop pressure-drop $\Delta P_1$ from the vessel inlet (15) to a distal end or outlet (13) of a vessel (10) using the formula:

$$\Delta P_1 = \Delta p_p + \sum_{i=1}^{m} \Delta p_s + \sum_{j=1}^{m-1} \Delta p_n + \Delta p_d$$

- Calculating the pressure drop ($\Delta p_p$) at a proximal part (18) of the vessel (10) using the Hagen-Poiseuille equation and assigning a volumetric flow rate of

$$Q_2 = CFR\ mL/s:$$

$$\Delta p_p = \frac{8\pi\mu L_p}{A_{ref}^2} Q_2$$

wherein $A_{ref} = \pi R_{ref}^2$ is the reference cross-sectional area of the vessel (10), and $L_p$ is the length of the proximal part (18) of the vessel (10).
- Calculating the pressure drop ($\Delta p_d$) at a distal part (16) of the vessel (10) using the Hagen-Poiseuille equation:

$$\Delta p_d = \frac{8\pi\mu L_d}{A_{ref}^2} Q_2$$

wherein $L_d$ is the length of the distal part (16) of the vessel (10).
- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q_2$$

wherein $L_n$ is the length of the normal part (22) of the vessel (10).
- Calculating the pressure drop ($\Delta p_s$) at a stenosed part (12) of the vessel (10) using the equation:

$$\Delta p_s = \frac{8\pi\mu L_s}{A_s^2} Q_2 + \frac{1.52}{2} \rho \left( \frac{1}{A_s} - \frac{1}{A_{ref}} \right)^2 Q_2^2$$

wherein $A_s$ is the minimum cross-sectional area at the stenosed part (12) of the vessel (10) and $L_s$ is the length of the stenosed part (12) of the vessel (10).

- Calculating the pressure drop pressure-drop $\Delta P_2$ from the vessel inlet (15) to a distal end (13) of the vessel (10) using the formula:

$$\Delta P_2 = \Delta p_p + \sum_{i=1}^{m} \Delta p_s + \sum_{j=1}^{m-1} \Delta p_n + \Delta p_d$$

- Calculating coefficients $a_v$ and $b_v$ of a general form of pressure drop in the stenosis (12) expressed as a $2 \times 2$ linear system in the form:

$$\Delta P = a_v Q + b_v Q^2$$

$$\Delta P_1 = a_v + b_v$$

$$\Delta P_2 = CFR \times a_v + CFR^2 \times b_v$$

- Plotting the pressure drop $\Delta P$ versus the volumetric flow rate $Q$, obtaining a $\Delta P$ - $Q$ curve.
- Calculating $P_d/P_a$ as:

$$\frac{P_d}{P_a} = 1 - \frac{a_v}{P_a} Q + \frac{b_v}{P_a} Q^2$$

- Plotting the ratio $P_d/P_a$ versus the flow Q in the coronary artery up to flow value equal to CFR mils and then calculating FFR by dividing the area-under-curve of the aforementioned plot by the maximum flow value CFR.

In a sixth embodiment of the invention, the method comprises:

- Acquiring a single two-dimensional X-Ray angiographic image (101).
- Applying computer algorithms for vessel delineation and calculating boundaries (31) of the vessel (10), then, computing a vessel centerline (30) and computing a length ($L_{vessel}$) and a diameter of the vessel (10) through the centerline (30) and the vessel boundaries (31), respectively. Then, computing the volume ($V_{vessel}$) of the vessel (10).
- Defining stenosed parts (12) (m in total) of the vessel (10). The vessel part between the inlet (15) and the first stenosed part (12) is determined as the proximal part (18) of the vessel (10). The vessel part between the end of the last stenosed part (12) and the end or outlet (13) of the vessel (10) is determined as the distal part (16) of the vessel (10). The remaining parts are determined as normal parts (22).
- Computing a reference diameter $D_{ref}$ using the formula:

$$D_{ref} = 2 \sqrt{\frac{V_{vessel}}{\pi L_{vessel}}}$$

- Computing a reference radius $R_{ref}$ as:

$$R_{ref} = \frac{D_{ref}}{2}$$

- Obtaining a minimum diameter $D_{min}$ in the stenosed parts (12) of the vessel (10).

- Computing using the geometrical data, reference diameter $D_{ref}$ and the minimum diameter $D_{min}$, the coronary flow reserve (CFR) given by Gould's formula

$$CFR = 3.7 - 0.01x + 0.00024x^2 - 0.000006x^3$$

wherein $x$ is the stenosis percentage defined as the ratio of the minimum diameter $D_{min}$ in the stenosed parts (12) of vessel (10) over the reference diameter $D_{ref}$ of the vessel (10).

- Calculating a pressure drop ($\Delta p_p$) at a proximal part (18) of the vessel (10) using the Hagen-Poiseuille equation and assigning a volumetric flow rate of

$$Q = CFR \ mL/s:$$

$$\Delta p_p = \frac{8\pi\mu L_p}{A_{ref}^2} Q$$

wherein $A_{ref} = \pi R_{ref}^2$ is a reference cross-sectional area of the vessel (10), and $L_p$ is a length of the proximal part (18) of the vessel (10).

- Calculating the pressure drop ($\Delta p_d$) at a distal part (16) of the vessel (10) using the Hagen-Poiseuille equation:

$$\Delta p_d = \frac{8\pi\mu L_d}{A_{ref}^2} Q$$

wherein $L_d$ is the length of the distal part (16) of the vessel (10).

- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q$$

wherein $L_n$ is the length of the normal part (22) of the vessel (10).

- Calculating the pressure drop ($\Delta p_s$) at a stenosed part (12) of the vessel (10) using the equation:

$$\Delta p_s = \frac{8\pi\mu L_s}{A_s^2} Q + \frac{1.52}{2} \rho \left( \frac{1}{A_s} - \frac{1}{A_{ref}} \right)^2 Q^2$$

wherein $A_s$ is the minimum cross-sectional area at the stenosed part (12) of the vessel (10) and $L_s$ is the length of the stenosed part (12) of the vessel (10).

- Calculating the pressure drop $\Delta P$ from the vessel inlet (15) to a distal end or outlet (13) of a vessel (10) using the formula:

$$\Delta P = \Delta p_p + \sum_{i=1}^{m} \Delta p_s + \sum_{j=1}^{m-1} \Delta p_n + \Delta p_d$$

In a seventh embodiment of the invention, the method comprises:

- Acquiring a single two-dimensional X-Ray angiographic image (101).
- Applying computer algorithms for vessel delineation and calculating boundaries (31) of the vessel (10), then, computing a vessel centerline (30); computing a length ($L_{vessel}$) and a diameter of a vessel (10) through the centerline (30) and the vessel boundaries (31), respectively; and, then, computing the volume ($V_{vessel}$) of the vessel (10).
- Defining stenosed parts (12) (m in total) of the vessel (10). The vessel part between the inlet (15) and the first stenosed part (12) is determined as the proximal part (18) of the vessel (10). The vessel part between the end of the last stenosed part (12) and the end or outlet (13) of the vessel (10) is determined as the distal part (16) of the vessel (10). The remaining parts are determined as normal parts (22).

- Computing a reference diameter $D_{ref}$ using the formula:

$$D_{ref} = 2\sqrt{\frac{V_{vessel}}{\pi L_{vessel}}}$$

- Computing a reference radius $R_{ref}$ as:

$$R_{ref} = \frac{D_{ref}}{2}$$

- Obtaining a minimum diameter $D_{min}$.
- Computing using the geometrical data, reference diameter $D_{ref}$ and the minimum diameter $D_{min}$, the coronary flow reserve (CFR) given by Gould's formula

$$CFR = 3.7 - 0.01x + 0.00024x^2 - 0.000006x^3$$

wherein $x$ is the stenosis percentage defined as the ratio of the minimum diameter $D_{min}$ in the stenosed parts (12) of vessel over the reference diameter $D_{ref}$ of the vessel (10).

- Computing a time needed by a contrast medium to reach certain landmarks of the vessel (10) (inlet (15) and outlet (13)

$$t_c = \frac{L_{vessel}}{u_h}$$

of the interrogated vessel (10)), by dividing the vessel length $L_{vessel}$ by the hyperaemic velocity $u_h$, wherein the hyperaemic flow velocity is a in the range of $u_h$ = 0.25 - 0.45 $m/s$, obtained from 88 patients in a retrospective study.

$$Q = \frac{V_{vessel}}{t_c}$$

- Assigning a volumetric flow rate of , computed by dividing the volume $V_{vessel}$ of the vessel (10) with the crossing time $t_c$.
- Calculating the pressure drop ($\Delta p_p$) at a proximal part (18) of the vessel (10) using the Hagen-Poiseuille equation:

$$\Delta p_p = \frac{8\pi\mu L_p}{A_{ref}^2} Q$$

$$A_{ref} = \pi R_{ref}^2$$

wherein is the reference cross-sectional area of the vessel (10), and $L_p$ is the length of the proximal part (18) of the vessel (10).

- Calculating a pressure drop ($\Delta p_d$) at a distal part (16) of the vessel (10) using the Hagen-Poiseuille equation:

$$\Delta p_d = \frac{8\pi\mu L_d}{A_{ref}^2} Q$$

wherein $L_d$ is the length of the distal part (16) of the vessel (10).

- Calculating the pressure drop ($\Delta p_n$) at a normal part (22) of the vessel (10) using the Hagen-Poiseuille equation:

$$\Delta p_n = \frac{8\pi\mu L_n}{A_{ref}^2} Q$$

wherein $L_n$ is the length of the normal part (22) of the vessel (10).

- Calculating the pressure drop ($\Delta p_s$) at a stenosed part (12) of the vessel (10) using the equation:

$$\Delta p_s = \frac{8\pi\mu L_s}{A_s^2}Q + \frac{1.52}{2}\rho\left(\frac{1}{A_s} - \frac{1}{A_{ref}}\right)^2 Q^2$$

wherein $A_s$ is the minimum cross-sectional area at the stenosed part (12) of the vessel (10) and $L_s$ is the length of the stenosed part (12) of the vessel (10).
- Calculating the pressure drop pressure-drop $\Delta P$ from the vessel inlet (15) to a distal end or outlet (13) of a vessel (10) using the formula:

$$\Delta P = \Delta p_p + \sum_{i=1}^{m}\Delta p_s + \sum_{j=1}^{m-1}\Delta p_n + \Delta p_d$$

In an eighth embodiment, the method comprises the steps of:

- Acquiring a single two-dimensional X-Ray angiographic image (101).
- Applying computer algorithms for vessel delineation and calculating boundaries (31) of the vessel (10), then, computing a vessel centerline (30) and computing a length ($L_{vessel}$) and a diameter of the vessel (10) through the centerline (30) and the vessel boundaries (31), respectively. Then, computing the volume ($V_{vessel}$) of the vessel (10).
- Defining stenosed parts (12) (m in total) of the vessel (10). The vessel part between the inlet (15) and the first stenosed part (12) is determined as the proximal part (18) of the vessel (10). The vessel part between the end or outlet (13) of the last stenosed part (12) and the end of the vessel (10) is determined as the distal part (16) of the vessel (10). The remaining parts are determined as normal parts (22).
- Computing a mean diameter $D_{p,mean}$ and a mean vessel area $A_{p,mean}$ of the proximal part (18) of the vessel (10).
- Computing a minimum diameter $D_{s,min}$ of the stenosed part(s) (12) of the vessel (10).
- Computing a mean diameter $D_{n,mean}$ of the normal parts (22) of the vessel (10) located between successive stenoses.
- Computing a mean diameter $D_{d,mean}$ of the distal part (16) of the vessel (10).
- Approximating a crossing time ($t_c$) by using a TIMI Frame Count (TFC) method and an image acquisition rate (fps) from angiography image information:

$$t_c = \frac{TFC}{fps}$$

- Computing a baseline volumetric flow rate ($Q_b$) by dividing the vessel volume ($V_{vessel}$) by the crossing time ($t_c$):

$$Q_b = \frac{V_{vessel}}{t_c}$$

- Computing, using the geometrical data, the coronary flow reserve (CFR) given by Gould's formula

$$CFR = 3.7 - 0.01x + 0.00024x^2 - 0.000006x^3$$

wherein $x$ is the stenosis percentage defined as the ratio of the minimum diameter in the stenosed parts (12) of vessel (10) over the diameter of a healthy (there is no stenosis) vessel (10).
- Computing a baseline velocity $u_b$ by diving the vessel length $L_{vessel}$ by the crossing time ($t_c$):

$$u_b = \frac{L_{vessel}}{t_c}$$

- Computing a hyperaemic velocity $u_h$ by multiplying the baseline velocity $u_b$ with *CFR:*

$$u_h = CFR \times u_b$$

- Calculating a pressure drop ($\Delta p_p$) at a proximal part (18) of the vessel (10) using the Hagen-Poiseuille equation and assigning a volumetric flow rate of

$$Q_p = u_h \times A_{p,mean}:$$

$$\Delta p_p = \frac{8\pi\mu L_p}{A_{p,mean}^2} Q_p$$

wherein $A_{p,mean} = \frac{\pi}{4} D_{p,mean}^2$ is the mean cross-sectional area at the proximal part (18) of the vessel (10), and $L_p$ is a length of the proximal part (18) of the vessel (10).

- Calculating the pressure drop ($\Delta p_d$) at a distal part (16) of the vessel (10) using the Hagen-Poiseuille equation and assigning a volumetric flow rate of

$$Q_d = u_h \times A_{d,mean}:$$

$$\Delta p_n = \frac{8\pi\mu L_d}{A_{d,mean}^2} Q_d$$

wherein $A_{d,mean} = \frac{\pi}{4} D_{d,mean}^2$ is the mean cross-sectional area at the distal part (16) of the vessel (10), $L_d$ is the length of the distal part (16) of the vessel (10).

- Calculating the pressure drop ($\Delta p_n$) at a normal part (22) of the vessel (10) using the Hagen-Poiseuille equation and assigning a volumetric flow rate of

$$Q_n = u_h \times A_{n,mean}:$$

$$\Delta p_n = \frac{8\pi\mu L_n}{A_{n,mean}^2} Q_n$$

wherein $A_{n,mean} = \frac{\pi}{4} D_{n,mean}^2$ is a mean cross-sectional area at the normal part (22) of the vessel (10), $L_n$ is the length of the normal part (22) of the vessel (10).

- Calculating the pressure drop ($\Delta p_s$) at a stenosed part (12) of the vessel (10) using the equation and assigning a volumetric flow rate of $Q_s = u_h \times A_s$:

$$\Delta p_s = \frac{8\pi\mu L_s}{A_s^2} Q_s + \frac{1.52}{2} \rho \left( \frac{1}{A_s} - \frac{1}{A_{p,mean}} \right)^2 Q_s^2$$

wherein $A_s = \frac{\pi}{4} D_s^2$ is the mean cross-sectional area at the stenosed part (12) of the vessel (10)), $A_{p,mean}$ is the mean cross-sectional area at the proximal part (18) of the vessel (10) and $L_s$ is the length of the stenosed part (12) of the vessel (10).

- Calculating the pressure drop pressure-drop $\Delta P$ from the vessel inlet (15) to a distal end or outlet (13) of the vessel (10) using the formula:

$$\Delta P = \Delta p_p + \sum_{i=1}^{m} \Delta p_s + \sum_{j=1}^{m-1} \Delta p_n + \Delta p_d$$

The invention has been described for coronary arteries, but it can also be applied to all vessels of the circulatory system.

The proposed method can also be applied for calculation of absolute blood flow rates under normal resting or simulated hyperaemic conditions.

The embodiments mentioned above are merely intended to be illustrative of the principles and effectives of the present disclosure and not to be limitations to the present disclosure. A person skilled in the art can make various changes or modifications to be above embodiments without departing from the scope of the claims. Therefore, all equivalent modifications or changes made by those with ordinary knowledge in the technical field without departing from the scope of the claims disclosed by the present disclosure should be considered as being covered by the invention.

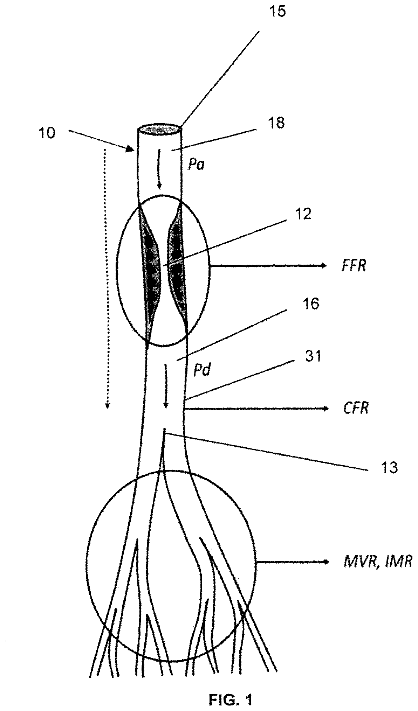

As described throughout the explanation of the embodiments of the invention, Figs. 1 to 4 illustrates steps of the method of the invention encompassed by the different embodiments described. Fig. 1 illustrates a vessel and its respective microcirculation highlighting where some indexes (CFR, FFR, MVR, IMR) are computed with the proposed invention, using a 2D angiographic image (101) and measuring crossing time $t_c$, vessel volume $V_{vessel}$ and flow rate $Q$ as explained before.

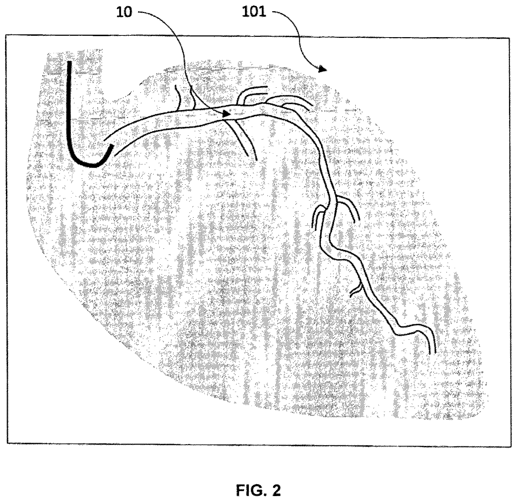

Fig. 2 shows a 2D angiographic image (101) with a coronary vessel tree and an interrogated coronary vessel (10).

Fig. 3 shows a geometrical model of the interrogated vessel (10) obtained from a 2D angiographic image (101). The geometrical model (20) for the coronary artery (10) with stenoses (12) is used to generate one or more clinical prognostic predictors according to the invention. Geometrical parameters of the model (20), such as the vessel inlet (15), the proximal part (18), the normal parts (22), the stenosed parts (12), the distal part (16), the vessel outlet (13), approximating the coronary vessel (10) are determined on the basis of a real 2D image (101) of said coronary vessel (10).

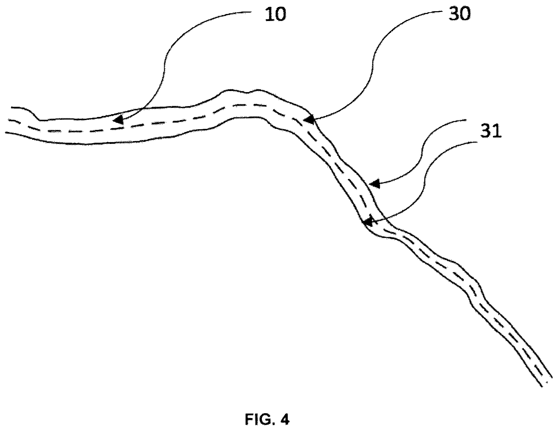

Fig. 4 shows an example of the vessel (10) with the vessel boundaries (31) defined (solid line) and the centerline (30) (dashed line).

Fig. 5 illustrates the radius of the vessel along the centerline (solid line) and the radius of the vessel along the centerline after extrapolation of an expected normal diameter (dashed line).

Herein is additionally included evidence highlighting the validity of the present invention and embodiments thereof in the form a study.

A pilot single-center analytical study designed to assess the diagnostic performance of FFR calculated using the method of the invention versus the gold-standard of FFR≤0.80 measured with a pressure-wire for the physiological assessment of intermediate vessel (10) stenoses has been carried out. Consecutive patients referred for diagnostic coronary angiography and potential revascularization between September 1st, 2020 and September 1st, 2022 were screened for eligibility based on pre-set inclusion and exclusion criteria as outlined below. Routine two-dimensional angiograms on optimal viewing angles were segmented to derive the vascular geometry of intermediate coronary lesions and nonlinear pressure-flow mathematical relationships were applied to compute FFR using the method of the invention.

**Inclusion Criteria:**

- Male or female subjects, > 18 years of age
- Patients with indication for coronary angiography other than an ACS.
- Intermediate vessel (10) stenosis with 30-70 percent diameter stenosis (%DS) and a reference vessel diameter of at least 2 mm by visual estimate
- Availability of at least one high-quality angiographic view of the vessel under interrogation

**Exclusion Criteria:**

- Known hypersensitivity or contraindications of receiving adenosine.
- Left Main (stenosis > 50%)
- Prior CABG, heart transplant or valve surgery, or prior TAVI/TAVR
- Severe heart failure (NYHA≥III)
- Severe aortic stenosis

- Low ejection fraction (<40 %)
- Arteries supplying akinetic or severe hypokinetic territories if already known based on prior imaging
- Diffuse CAD defined as the presence of diffuse, serial gross luminal irregularities present across most of the coronary trees or tandem lesions.

The primary study hypothesis was that FFR calculated using the method of the invention has good correlation with the invasive FFR reference test and superior diagnostic performance compared to two-dimensional Quantitative Coronary Angiography (2DQCA; % diameter stenosis $\geq$50%) to predict FFR$\leq$0.80 for the identification of physiologically significant lesions. Secondary endpoints included feasibility of FFR computation using the method of the invention from a single-view angiographic image in FFR interrogated vessels, and time of image processing and computation for calculation of FFR using the method of the invention .

Categorical baseline patient demographics, clinical variables and angiographic vessel characteristics are presented as count and percentages. Continuous variables are presented as means and standard deviation if originating from a normal distribution or reported as medians and inter-quartile range if non-normally distributed. Baseline clinical variables were analyzed on a per-patient basis and angiographic lesion characteristics and FFR results on a per-vessel basis. An FFR calculated using the method of the invention value $\leq$0.80 is scored as positive implying a hemodynamically critical stenosis, whereas an FFR calculated using the method of the invention value >0.8 is negative and would defer revascularization. The FFR calculated using the method of the invention diagnostic accuracy was assessed with the Area under-the-curve (AUC) of the Receiver-operating characteristic (ROC) analysis using invasive FFR as the reference test. A cut-off value of FFR $\leq$0.80 was applied to identify functional stenoses. The vessel based AUCs for the FFR calculated using the method of the invention and 2DQCA models were compared with the non-parametric DeLong test.

By taking FFR as the reference solution, we report diagnostic classification indexes including accuracy, sensitivity, specificity, positive predictive value (PPV) and negative predictive value (NPV) for the FFR computed using the method of the invention values to detect a physiologically significant stenosis on the cut-off value of $\leq$0.80. False negative ratio and false positive ratio are reported along with likelihood ratios. All diagnostic outcomes are dichotomously scored per target vessel in comparison with invasive FFR. Pearson correlation coefficient r was computed between FFR calculated using the method of the invention and invasive FFR. A Bland-Altman analysis with 95% agreement levels was performed to present the agreement between FFR calculated using the method of the invention and FFR reference.

**Table 1. Baseline clinical and angiographic variables**

| | |
|---|---|
| Age (years) | 65.8±10.6 |
| Male gender | 66/88 (75.0%) |
| Hyperlipidemia | 68/88 (77.3%) |
| Smoking | 59/88 (67.0%) |
| Diabetes | 20/88 (22.7%) |
| Hypertension | 73/88 (83.0%) |
| Family CAD history | 13/88 (14.8%) |
| Previous PCI intervention | 28/88 (31.8%) |
| Previous CABG surgery | 0/88 (0%) |
| Stable angina | 18/88 (20.5%) |
| Unstable angina | 20/88 (22.7%) |
| Chronic CCS scale (0-IV) | 0 (0-0) |
| Ejection fraction (%) | 53.2±9.8 |
| Radial access | 88/88 (100%) |
| Adenosine intravenous | 88/88 (100%) |
| Vessel bifurcations | 24/88 (27.3%) |
| Tandemlesions | 10/88 (11.4%) |
| LAD | 74/88 (84.1%) |
| RCA | 9/88 (10.2%) |
| LCX | 5/88 (5.7%) |

(continued)

| Minimum lumen diameter | 1.76±0.48 mm |
|---|---|
| Reference vessel diameter | 3.25±0.56 mm |
| Percent (%) diameter stenosis | 45.7±11.0% |

A total of 88 consecutive patients with a single intermediate vessel (10) lesion were analyzed. Overall, three vessels were excluded, two with severe tortuosity and 1 case with poor contrast quality precluding image analysis for FFR computation using the method of the invention. Therefore, overall feasibility of FFR computation using the method of the invention was 88/91 (96.7%). Baseline variables and clinical characteristics of the patients were typical of chronic vessel (10) disease and are outlined in the table above. Lesions were located primarily in the left anterior descending artery (LAD; n = 74) and 9 cases in the right vessel (10) (RCA) and 5 in the left circumflex artery (LCX). Radial access and peripheral intravenous infusion of adenosine was applied in all cases. Lesions were located at coronary bifurcations in 27 cases (30.7%) and tandem stenoses were found in 10 arteries (11.4%; n = 7 with 2 lesions and n = 3 with 3 lesions). Quantitative coronary angiographic analysis documented a minimum lumen diameter of 1.76±0.48 mm with an average percent (%) diameter stenosis of 45.7±11.0%. Mean arterial pressure at rest was 93.9±7.0 mm Hg and measured pressure-wire FFR was 0.82±0.07. Abnormal FFR ≤0.80 was measured in 28 cases (31.8%).

The FFR computed using the method of the invention was on average 0.84±0.12 and correlated well with invasive FFR (r = 0.67, p<0.001). There was good agreement between FFR calculated using the method of the invention and invasive FFR (mean difference: 0.02±0.09) - Figures 8 and 9. Applying the FFR cut-off value of ≤0.80 to FFR calculated using the method of the invention resulted in 22 true positives, 56 true negatives, 4 false positives, and 6 false negatives. FFR calculated using the method of the invention ≤0.80 predicted the measured FFR≤0.80 with a sensitivity of 78.6%, specificity of 93.3%, positive likelihood ratio of 11.8, and negative likelihood ratio of 0.23. Overall diagnostic accuracy of FFR calculated using the method of the invention for diagnosing a critical epicardial artery stenosis as defined by FFR≤0.80 was 88.6% (78 cases classified correctly out of 88 in total). A summary of all diagnostic statistics with their corresponding 95% confidence intervals is included in Table 2. FFR calculated using the method of the invention had superior discriminatory capacity compared to percent diameter stenosis at the 50% threshold in diagnosing physiologically significant intermediate vessel (10) stenoses using the FFR cut-off value of ≤0.80. On ROC analysis, FFR calculated using the method of the invention had a significantly higher area under the curve (0.95 [95% CI: 0.54 - 1.00]) than 50%DS on 2D-QCA (0.71 [95% CI: 0.59 - 0.82]; p < 0.001). Corresponding ROC curves are shown in Figure 10. Median time of image processing was 2 minutes and median time of computation of FFR using the method of the invention was 0.1 seconds.

**Table 2. Diagnostic performance statistics**

| Statistical test | Value | 95% CI |
|---|---|---|
| Sensitivity | 78.6% | 59.0% - 91.7% |
| Specificity | 93.3% | 83.8% - 98.2% |
| Positive Likelihood Ratio | 11.8 | 4.48 - 30.98 |
| Negative Likelihood Ratio | 0.23 | 0.11 - 0.47 |
| Disease prevalence | 31.8% | 22.3% - 42.6% |
| Positive Predictive Value | 84.6% | 65.1% - 95.6% |
| Negative Predictive Value | 90.3% | 80.1% - 96.4% |
| Accuracy | 88.6% | 80.0% - 94.4% |
| ROC area under the curve | 95 % | 54% - 100% |

From these results, it has been concluded that FFR calculated using the method of the invention may derive an image-based, simple, and quick metric of fractional flow reserve with high diagnostic accuracy based on a single two-dimensional coronary angiographic image.

**Claims**

1. A computer-implemented method for calculating a pressure drop along one or more vessels comprising one or more stenosis, the method comprising the steps of:

   - obtaining at least one two-dimensional image (101) of the vessels (10);
   - obtaining a centerline (30) and a boundary (31) of each vessel (10) by using image processing algorithms;
   - calculating a length, volume and/or a diameter of each vessel (10) by using the centerline (30) and the boundary (31) obtained;
   - obtaining a baseline or a hyperaemic volumetric flow ($Q$);
   - determining a set of stenosed parts (12);
   - defining in the vessel (10) a proximal part (18), between an inlet (15) and a first stenosed part (12), a distal part (16), between a last stenosed part (12) and an outlet (13), and a set of normal parts (22), between two stenosed parts (12);
   - calculating a pressure drop at a proximal part (18) and a pressure drop at a distal part (16), respectively, as:

   $$\Delta p_p = \frac{8\pi\mu L_p}{A_{p,ref}^2} Q$$

   $$\Delta p_d = \frac{8\pi\mu L_d}{A_{d,ref}^2} Q$$

   wherein $A_{p,ref}$ being the reference cross-sectional area at the proximal part (18) of the vessel, $L_p$ being the length of the proximal part (18) of the vessel (10), $A_{d,ref}$ being the reference cross-sectional area at the distal part (16) of the vessel (10) and $L_d$ being the length of the distal part (16) of the vessel (10), are previously obtained from two-dimensional images;

   - calculating a pressure drop at normal parts (22) and a pressure drop at stenosed parts (12), respectively, as:

   $$\Delta p_n = \frac{8\pi\mu L_n}{A_{n,ref}^2} Q$$

   $$\Delta p_s = \frac{8\pi\mu L_s}{A_s^2} Q + \frac{1.52}{2} \rho \left(\frac{1}{A_s} - \frac{1}{A_{p,ref}}\right)^2 Q^2$$

   wherein $A_{n,ref}$ being the reference cross-sectional area at the normal part (22) of the vessel (10), $L_n$ being the length of the normal part (22) of the vessel (10), $A_s$ being the minimum cross-sectional area at the stenosed part (12) of the vessel (10) and $L_s$ being the length of the stenosed part (12) of the vessel (10), are previously obtained from two-dimensional images; and

   - calculating a total pressure drop $\Delta P$ from the inlet (15) to the outlet (13) of the vessel (10) using the formula:

   $$\Delta P = \Delta p_p + \sum_{i=1}^{m} \Delta p_s + \sum_{j=1}^{m-1} \Delta p_n + \Delta p_d$$

2. The computer-implemented method according to claim 1, wherein the two-dimensional image (101) is obtained from a time-series X-Ray angiography, and further comprising steps of calculating a volume $V_{vessel}$ of each vessel (10) by using the length $L_{vessel}$ and calculating a diameter and approximating a crossing time ($t_c$) using a TIMI Frame Count (TFC) method by defining the number of cine-frames required for contrast médium to pass from inlet (15) to outlet (13) in the vessel (10), and wherein the baseline volumetric flow ($Q_b$) is calculated by using the volume $V_{vessel}$ of each vessel (10) and the crossing time ($t_c$) as:

   $$Q_b = V_{vessel}/t_c$$

3. The computer-implemented method according to claim 1, wherein the step of calculating a baseline volumetric flow ($Q_b$) is performed by using a power-law formulae:

$$Q_b = aX^b$$

wherein $X$ is a morphological parameter, which is the diameter, the length or an axi-symmetric volume of the vessel (10), $a$ is a constant calculated by fitting regression models on previously analyzed experimental or human data previously known, and $b$ is a fitted power-law exponent based on experimental or human data previously known that is preferably in the range of 0.6 - 0.8 as in paper_Huo Y and Kassab G S entitled "*A Scaling Law of Vascular Volume*" (Biophysical Journal Volume 96 January 2009 347-353)

4. The computer-implemented method according to any of claims 1 to 3, further comprising steps of:

- calculating a volume $V_{vessel}$ of each vessel (10) by using the length $L_{vessel}$ and diameter;
- assigning a constant hyperaemic or baseline velocity ($U$) for the blood flow in the vessel (10);
- computing a crossing time ($t_c$) using the formula:

$$t_c = L_{vessel}/U$$

and wherein the hyperaemic volumetric flow ($Q_h$) or the baseline volumetric flow ($Q_b$) is calculated by using the volume $V_{vessel}$ of each vessel (10) and the crossing time ($t_c$) as:

$$Q = V_{vessel}/t_c$$

5. The computer-implemented method according to claim 4, wherein the hyperaemic flow velocity is a constant value in the range of $u_h$ = 0.25 - 0.45 $m/s$.

6. The computer-implemented method according to claim 4, wherein the baseline flow velocity is a constant value in the range of $u_b$ = 0.14 - 0.16 $m/s$.

7. The computer-implemented method according to claim 4, wherein the pressure $P_d$ at the outlet of the vessel is calculated by a numerical method, using a two-dimensional axisymmetric vessel (10) grid model, a continuity equation and a Navier-Stokes equation:

$$\nabla \cdot U = 0$$

$$\rho \left( \frac{\partial U}{\partial t} + (U \cdot \nabla)U \right) = -\nabla p + \mu \nabla^2 U$$

wherein, $U$ is a blood flow velocity, $p$ is a pressure, $\rho$ is a blood density, and $\mu$ is a blood viscosity; and wherein the mean arterial pressure ($P_a$) is an inlet (15) boundary condition, and the hyperaemic volumetric flow rate ($Q_h$) is an outlet (13) boundary condition.

8. The computer-implemented method according to any of claims 1 to 7, wherein the volumetric flow ($Q$) is a volumetric flow rate at hyperaemia ($Q_h$) calculated by following the stages of:

- calculating a baseline volumetric flow ($Q_b$);
- obtaining a minimum diameter in the stenosed parts (12) and a reference diameter in the proximal part of the vessel (10);
- calculating a coronary flow reserve (CFR) as:

$$CFR = 3.7 - 0.01x + 0.00024x^2 - 0.000006x^3$$

wherein $x$ is the stenosis percentage, defined as the ratio of the minimum diameter in the stenosed parts (12) of vessel (10) over the reference diameter in the proximal part (18);
- calculating the volumetric flow rate at hyperaemia ($Q_h$) as:

$$Q_h = CFR \times Q_b$$

9. The computer-implemented method according to claim 1, wherein the baseline flow $Q_b$ is equal to 1mL/s and the hyperaemic volumetric flow ($Q_h$) is numerically equal to a coronary flow reserve (CFR) calculated by following the stages of:

   - obtaining a minimum diameter in the stenosed parts (12) and a maximum diameter in the proximal part (18) of the vessel;
   - calculating a coronary flow reserve (CFR) as:

$$CFR = 3.7 - 0.01x + 0.00024x^2 - 0.000006x^3$$

   wherein $x$ is the stenosis percentage, defined as the ratio of the minimum diameter in the stenosed parts (12) of vessel (10) over the maximum diameter.

10. The computer-implemented method according to claim 1, wherein the steps of calculating a pressure drop at a proximal part (18) and a pressure drop at a distal part (16), calculating a pressure drop at normal parts (22) and a pressure drop at stenosed parts (12) and calculating a total pressure drop $\Delta P$ are performed twice, assigning a first volumetric flow ($Q_1$) and obtaining a first total pressure drop $\Delta P_1$ and assigning a second volumetric flow ($Q_2$) and obtaining a second total pressure drop $\Delta P_2$, and further comprising a step of calculating a fractional flow reserve (FFR) by following the stages of:

    - defining the total pressure drop $\Delta P$ as:

$$\Delta P = a_v Q + b_v Q^2$$

    - obtaining parameters (a, b) by solving the equation system:

$$\Delta P_1 = a_v \cdot Q_1 + b_v \cdot Q_1^2$$

$$\Delta P_2 = a_v \cdot Q_2 + b_v \cdot Q_2^2$$

    - calculating $P_d/P_a$ as:

$$\frac{P_d}{P_a} = 1 - \frac{a_v}{P_a} Q + \frac{b_v}{P_a} Q^2$$

    - plotting the ratio $P_d/P_a$ versus the flow Q in the coronary artery up to flow value 4ml/s and then calculating FFR by dividing the area-under-curve of the aforementioned plot by the maximum flow value 4.

11. The computer-implemented method according to any of claims 1 to 10, further comprising a step of obtaining a mean aortic pressure (MAP) in rest state, obtaining the baseline pressure at the inlet (15) ($P_a$), calculating pressure at the distal part (16) ($P_d$) as:

$$P_d = P_a - \Delta P$$

and calculating a fractional flow reserve (FFR) as:

$$FFR = P_d/P_a$$

12. The computer-implemented method according to any of claims 1 to 11, further comprising a step of calculating a hyperaemic stenosis resistance (HSR), an absolute microvascular resistance (MVR) and an index microvascular resistance (IMR) as follows, respectively:

$$HSR = \frac{\Delta P}{Q_h}$$ (divided by hyperaemic flow $Q_h$) or alternatively $$HSR = \frac{\Delta P}{U}$$ (divided by Velocity U at hyperaemic state)

$$MVR = \frac{Pa - \Delta P}{Q_h} \text{ or } MVR = \frac{Pd}{Q_h}$$

$$IMR = \frac{Pa - \Delta P}{CFR} \cdot t$$

13. The computer-implemented method according to any of claims 1 to 12, wherein the two-dimensional image (101) is obtained from X-Ray angiography.

14. The computer-implemented method according to any of claims 1 to 12, wherein the two-dimensional image (101) is obtained from computed tomography.

15. The computer-implemented method according to any of claims 1 to 12, wherein the two-dimensional image (101) is obtained from magnetic resonance angiography.

16. A system for calculating a pressure drop along one or more vessels comprising:

    - an X-Ray angiography device for obtaining at least one two-dimensional image (101) of the vessels (10); and
    - a processing unit configured for performing the steps of the method according to claim 13.

17. A system for calculating a pressure drop along one or more vessels comprising:

    - a computed tomography device for obtaining at least one two-dimensional image (101) of the vessels (10); and
    - a processing unit configured for performing the steps of the method according to claim 14.

18. A system for calculating a pressure drop along one or more vessels comprising:

    - a magnetic resonance angiography device for obtaining at least one two-dimensional image (101) of the vessels (10); and
    - a processing unit configured for performing the steps of the method according to claim 15.

**FIG. 1**

FIG. 2

FIG. 3

FIG. 4

**FIG. 5**

Obtaining at least one two-dimensional image of the coronary vessels

obtaining a centerline and a boundary of each coronary vessel

calculating a length, volume and/or a diameter of each vessel

obtaining a baseline or a hyperaemic volumetric flow (Q)

determining a set of stenosed parts

defining in the vessels a proximal part, a distal part, normal parts and stenosed parts

calculating a pressure drop at a proximal part, a pressure drop at a distal part, a pressure drop at normal parts and a pressure drop at stenosed parts

calculating a total pressure drop ΔP from the inlet to the outlet of the vessel

Calculating a fractional flow reserve (FFR), a hyperaemic stenosis resistance (HSR), an absolute microvascular resistance (MVR) and/or an index microvascular resistance (IMR)

**FIG. 6**

Europäisches
Patentamt

European
Patent Office

Office européen
des brevets

# EUROPEAN SEARCH REPORT

**Application Number**

EP 23 38 6107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/200867 A1 (LAVI IFAT [IL] ET AL) 17 July 2014 (2014-07-17) <br> * paragraph [0240] - paragraph [0390] * <br> * figures 1-12 * | 1-18 | INV. <br> A61B5/02 |
| Y | LYRAS KONSTANTINOS G. ET AL: "An improved reduced-order model for pressure drop across arterial stenoses", PLOS ONE, vol. 16, no. 10, 1 October 2021 (2021-10-01), page e0258047, XP093053367, DOI: 10.1371/journal.pone.0258047 <br> * the whole document * | 1-18 | |
| Y | GOSLING REBECCA C ET AL: "Effect of side branch flow upon physiological indices in coronary artery disease", JOURNAL OF BIOMECHANICS, PERGAMON PRESS, NEW YORK, NY, US, vol. 103, 26 February 2020 (2020-02-26), XP086118213, ISSN: 0021-9290, DOI: 10.1016/J.JBIOMECH.2020.109698 [retrieved on 2020-02-26] <br> * the whole document * | 1-18 | |
| Y | US 2015/065864 A1 (SHARMA PUNEET [US] ET AL) 5 March 2015 (2015-03-05) <br> * paragraph [0025] - paragraph [0044] * <br> * figures 1-4 * | 1-18 | **TECHNICAL FIELDS SEARCHED (IPC)** <br><br> A61B |
| A | US 2021/338088 A1 (BOUWMAN CHRIS [NL] ET AL) 4 November 2021 (2021-11-04) <br> * paragraph [0116] - paragraph [0184] * <br> * figures 1-16 * | 1-18 | |

-/--

The present search report has been drawn up for all claims

| Place of search | Date of completion of the search | Examiner |
|---|---|---|
| Berlin | 12 September 2024 | Abraham, Volkhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches Patentamt
European Patent Office
Office européen des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Y. HUO ET AL: "A validated predictive model of coronary fractional flow reserve", AJP: HEART AND CIRCULATORY PHYSIOLOGY, vol. 18, no. 1, 23 November 2011 (2011-11-23), pages 518-1338, XP055074016, ISSN: 0363-6135, DOI: 10.1098/rsif.2011.0605 * the whole document * | 1-18 | |
| A | STERGIOPULOS N ET AL: "Computer simulation of arterial flow with applications to arterial and aortic stenoses", JOURNAL OF BIOMECHANICS, PERGAMON PRESS, NEW YORK, NY, US, vol. 25, no. 12, 1 December 1992 (1992-12-01), pages 1477-1488, XP022917419, ISSN: 0021-9290, DOI: 10.1016/0021-9290(92)90060-E [retrieved on 1992-12-01] * the whole document * | 1-18 | |

TECHNICAL FIELDS SEARCHED (IPC)

The present search report has been drawn up for all claims

| Place of search | Date of completion of the search | Examiner |
|---|---|---|
| Berlin | 12 September 2024 | Abraham, Volkhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 38 6107

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2024

| Patent document cited in search report | Publication date | Patent family member(s) | | Publication date |
|---|---|---|---|---|
| US 2014200867 A1 | 17-07-2014 | CN | 105190630 A | 23-12-2015 |
| | | EP | 2946319 A1 | 25-11-2015 |
| | | EP | 2946321 A1 | 25-11-2015 |
| | | EP | 3753494 A1 | 23-12-2020 |
| | | JP | 6542129 B2 | 10-07-2019 |
| | | JP | 6636331 B2 | 29-01-2020 |
| | | JP | 6790179 B2 | 25-11-2020 |
| | | JP | 7039442 B2 | 22-03-2022 |
| | | JP | 2016509501 A | 31-03-2016 |
| | | JP | 2016511649 A | 21-04-2016 |
| | | JP | 2019088792 A | 13-06-2019 |
| | | JP | 2019193808 A | 07-11-2019 |
| | | JP | 2021045558 A | 25-03-2021 |
| | | JP | 2022169579 A | 09-11-2022 |
| | | KR | 20150110609 A | 02-10-2015 |
| | | US | 2014200867 A1 | 17-07-2014 |
| | | US | 2017364658 A1 | 21-12-2017 |
| | | US | 2018268941 A1 | 20-09-2018 |
| | | US | 2019164649 A1 | 30-05-2019 |
| | | US | 2019385745 A1 | 19-12-2019 |
| | | WO | 2014111927 A1 | 24-07-2014 |
| | | WO | 2014111929 A1 | 24-07-2014 |
| | | WO | 2014111930 A1 | 24-07-2014 |
| US 2015065864 A1 | 05-03-2015 | NONE | | |
| US 2021338088 A1 | 04-11-2021 | CN | 112543618 A | 23-03-2021 |
| | | EP | 3806742 A1 | 21-04-2021 |
| | | JP | 7314183 B2 | 25-07-2023 |
| | | JP | 7531020 B2 | 08-08-2024 |
| | | JP | 2021528137 A | 21-10-2021 |
| | | JP | 2023109889 A | 08-08-2023 |
| | | US | 2019380593 A1 | 19-12-2019 |
| | | US | 2021338088 A1 | 04-11-2021 |
| | | WO | 2019238754 A1 | 19-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3763285 A1
- US 9858387 B2

- WO 2019238754 A1

**Non-patent literature cited in the description**

- **N H PIJLS** ; **J A VAN SON** ; **R L KIRKEEIDE** ; **B DE BRUYNE** ; **K L GOULD**. Experimental basis of determining maximum coronary, myocardial, and collateral blood flow by pressure measurements for assessing functional stenosis severity before and after percutaneous transluminal coronary angioplasty''. *Circulation*, 1993, vol. 87 (4), 1354-1367

- **HUO Y** ; **SVENDSEN M** ; **CHOY J S** ; **ZHANG Z.-D** ; **KASSAB G S**. A validated predictive model of coronary fractional flow reserve. *J. R. Soc. Interface*, 2012, vol. 9, 1325-1338
- **GOULD, K LIPSCOMB** ; **G W HAMILTON**. Physiologic basis for assessing critical coronary stenosis. Instantaneous flow response and regional distribution during coronary hyperemia as measures of coronary flow reserve. *The American Journal of Cardiology*, 1974, vol. 33 (1), 87-94